# EUROPEAN PATENT SPECIFICATION

(11) **EP 1 537 132 B1**
(45) Date of publication and mention of the grant of the patent: **04.01.2006**
(21) Application number: 03787894.9
(22) Date of filing: 14.08.2003
(51) Int. Cl.: C07H 17/00, A61K 31/70

(54) **SALT OF MORPHINE-6-GLUCURONIDE**
SALZ VON MORPHIN-6-GLUCURONID
SEL DE MORPHINE-6-GLUCURONIDE

(30) Priority: 14.08.2002 GB 0218811
(43) Date of publication of application: 08.06.2005
(73) Proprietor: CeNes Limited, Cambridge CB4 9ZR (GB)
(72) Inventor: GRAHAM, John Aitken, c/o CeNeS Limited, Cambridge CB4 9ZR (GB); FRANZMAIR, Rudolf, 8600 Bruck/Mur (AT)
(74) Representative: Thornton, Neil
(86) International application number: PCT/GB2003/003562
(87) International publication number: WO 2004/016633

(56) References cited:
- EP-A- 0 816 375
- WO-A-95/20966
- WO-A-99/15528

## Description

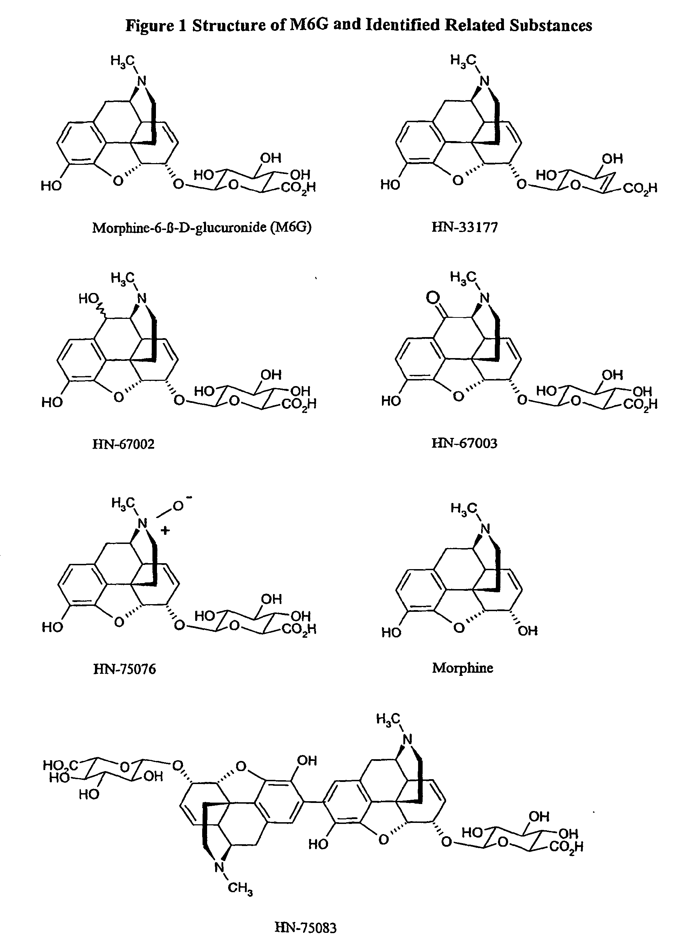

This invention relates to a salt of morphine-6-β-D-glucuronide (M6G; see Figure 1) with improved stability, and to use of the salt as a medicament, in particular as an analgesic.

M6G is a metabolite of morphine which is known to be a more powerful analgesic than morphine itself and yet has fewer side effects. Methods of preparation of M6G are described in WO 93/03051, WO 93/05057, WO 99/58545 and WO 99/38876.

Whilst M6G base is stable when stored at -20°C, it does degrade when stored at room temperature. This degradation is not only noted by an increase in detectable degradation products, but also by a marked colour change of the compound. This will limit the shelf life of M6G base at ambient temperature.

It has now been found that the hydrobromide salt of M6G (M6G.HBr) is surprisingly stable compared to M6G base and other M6G salts, in particular the hydrochloride (M6G.HCl) and sulphate (M6G₂.H₂SO₄) salts. M6G.HBr showed a very limited amount of degradation and no discolouration after storage at room temperature for six years (see Example 1 below).

According to the invention there is provided a hydrobromide salt of M6G (M6G.HBr). Methods of preparation of M6G.HBr are described in Examples 2 and 3 below.

M6G.HBr may be used as a medicament, in particular as an analgesic. Examples are for the treatment of moderate to severe, acute and chronic nociceptive pain (such as post-operative pain, pain associated with malignant and non-malignant diseases), and neuropathic pain.

M6G.HBr may be administered by any suitable route. Examples are as a solid formulation (e.g. for oral, dry powder inhalation), as a solution formulation (e.g. intravenous (including infusion for PCA), subcutaneous, intranasal, or sublingual), or as a transdermal formulation (e.g. by simple diffusion or by enhanced electrophoretic methods). Transdermal administration of pharmaceutically acceptable acid addition salts of M6G is described in US 5,705,186.

According to the invention there is also provided a pharmaceutical composition comprising an analgesically effective amount of M6G.HBr together with a pharmaceutically acceptable carrier, excipient, or diluent.

An analgesically effective amount of M6G.HBr will vary with the route of administration, and with factors such as the age, sex, weight, and condition of the subject being administered, and with the type of condition being treated. In general, a suitable dose for an acute condition will be lower than for a chronic condition.

A suitable dose is in the range of 1-1000mg/70Kg, preferably 1-200mg/70Kg, more preferably in the range of 5-75mg/70Kg. A preferred dose for acute use is in the range of 5-75mg/70Kg. A preferred dose for chronic use is in the range of 30-500mg/kg. Dosage for routes of administration where bio-availability is high (e.g. intravenous, subcutaneous, intranasal, sublingual) will be lower than for routes with low bio-availability (e.g. oral).

M6G.HBr may also be used for the symptomatic treatment of breathlessness in patients with advanced cancer. Any suitable route of administration may be used, but a preferred route is inhalation of nebulized M6G.HBr. The effect of administration of nebulized M6G is described by Quigley *et al* (in *J. Pain Symptom* Manage., Letters, Vol 23, No.1 (2002), pages 7-9). A dosage of M6G.HBr effective for the treatment of breathlessness in a subject with advanced cancer will vary with the route of administration, and with factors such as the age, sex, weight, and condition of the subject being administered. A suitable dose is in the range of 1-200mg/70Kg, preferably in the range of 5-75mg/70Kg.

There is further provided according to the invention a method of making M6G.HBr which comprises: (i) contacting a hydrogen bromide solution with a solution of M6G in methanol; (ii) contacting the solution resulting from step (i) with an organic solvent to precipitate M6G.HBr; and (iii) isolating M6G.HBr precipitated in step (ii).

Preferably the solutions and solvent are at -15°C, or below. This minimises formation of degradation products.

Preferably the precipitated M6G.HBr is washed to minimise the amount of organic solvent present. For example, the precipitated M6G.HBr may be washed with diethyl ether.

A preferred organic solvent is 2-propanol.

According to a preferred method a cooled diluted solution of HBr is added to a continuously stirred, cooled (to at least -15°C) solution of M6G in methanol. Then 2-propanol (or other suitable organic solvent) is added, and the resulting suspension is maintained below -15°C, while continuously stirring. Following stirring of the suspension the resultant crystals are filtered and washed with a suitable solvent (e.g. 2-propanol or diethyl ether) and dried by suitable means (e.g. under vacuum at room temperature).

The following examples 1 and 2 relate to the stability of M6G salts at room temperature, and methods of preparation of M6G salts, respectively. Table 1 shows the stability data for the M6G salts tested, and Figure 1 shows the chemical structure of M6G and identified degradants. Example 3 relates to the stability of M6G salts and base at 25°C/60%RH, 40°C/75%RH and 60°C. Tables 2-4 show the data relating to example 3.

### Example 1 Stability of M6G salts at room temperature over 6 years

### Analytical investigation by HPLC:

Samples of the hydrochloride salt (M6G.HCl) (205-2056), the sulphate salt (M6G₂.H₂SO₄) (205-2060), and the hydrobromide salt (M6G.HBr) (205-2059) of M6G were stored at room temperature for almost 6 years and then analysed by HPLC. The results are shown in Table 1, together with the results of HPLC analysis of samples prepared under similar conditions a few months earlier.

### Results:

M6G.HCl (205-2056): The content of M6G decreased to 69% (starting from -82%). HN-67002 and HN-67003 (which are typically oxidation products) increased to 1.3% and 2.1% respectively. The content of HN-33177, a synthetic impurity of M6G, remained unchanged. However, there are 17 peaks present in the chromatogram that cannot be identified by retention time. The total of these impurities is 9.2 area %.

M6G₂.H₂SO₄ (205-2060): The content of M6G decreased to 63% (starting from -77%). HN-67002 and HN-67003 increased to 1.1% and 1.8% respectively. The content of HN-33177 did not change. However, there are 13 peaks present in the chromatogram that cannot be identified by retention time. The total of these impurities is 10.7 area % with a dominant peak at 23.5 min (6.55 area %).

M6G.HBr (205-2059): The content of M6G did not decrease at all and the content of HN-67002 (0.5%) and HN-67003 (0.2%) is much lower than in the samples discussed above. There are only 4 additional peaks present in the chromatogram. None of these are bigger than 0.4 area %. The result is superior to the two other salts tested.

### Conclusion:

The hydrobromide salt of M6G shows very limited degradation and was not discoloured after storage for six years at room temperature compared to the free base and other salts investigated. Thus, the hydrobromide salt of M6G has improved stability at room temperature compared to the hydrochloride and sulphate salts of M6G.

### Example 2 Preparation of hydrobromide and sulphate salts of M6G

### Preparation of Q 3196 (M6G.HBr, 304-4428):

4.99g of M6G.2H₂O were dissolved in 11ml of Methanol and cooled to -15°C. 1.16ml of HBr (48% in water) was diluted with 0.85ml of Methanol and cooled to -15°C and added slowly to the solution of M6G. A clear, highly viscous, pale yellow solution was obtained. The solution was stirred for 5 minutes before 100ml 2-propanol (-15°C) were added. The product precipitated immediately. The slurry was stirred for 3.5 hours at -20°C, the crystals were filtered off, washed with 37.5ml cold 2-propanol (-20°C) and dried at room temperature in a high vacuum. The yield was 5.61g.

### Preparation of Q 3195 (M6G₂.H₂SO₄, 304-4429) :

5.02g of M6G.2H₂O were dissolved in 11ml of Methanol and cooled to -15°C. 0.35ml of H₂SO₄ (96%) was diluted with 0.85ml of Methanol and cooled to -15°C and added slowly to the solution of M6G. A clear, highly viscous, pale yellow solution was obtained. The solution was stirred for 5 minutes before 100ml 2-propanol (-15°C) were added. The product precipitated immediately. The slurry was stirred for 3.5 hours at -20°C, the crystals were filtered off, washed with 37.5ml cold 2-propanol (-20°C) and dried at room temperature in a high vacuum. The yield was 5.36g.

### Example 3 Stability of M6G salts after 1 month at 60°C and 3 months at 25°C/60% relative humidity and 40°C/75% relative humidity

The analytical data below gives clear evidence that the stability of the hydrobromide salt is superior to all other salts assessed and in addition would appear to be more stable than Morphine-6-glucuronide base. The data demonstrates that the hydrobromide salt is stable when subjected to storage conditions of 25°C/60%RH and 40°C/75%RH for 3 months and 60°C for 1 month. The base appears to be relatively stable to storage conditions of 25°C/60%RH after three months, but shows signs of degradation at 40°C/75%RH over 3 months and 60°C over 1 month.

All of the other salts show some form of degradation at 25°C/60%RH and at elevated temperature and humidity.

The Morphine-6-glucuronide sulphate salt is the least stable at 25°C/60%RH, whilst the Morphine-6-glucuronide hydrochloride is the least stable at 40°C/75%RH as this shows the greatest level of degradation of all the salts.

### Introduction

Various salts and the base of Morphine-6-glucuronide have been subjected to storage conditions of 25°C/60%RH and 40°C/75%RH for 3 months and 60°C for 1 month.

The analytical testing comprised of:
- Visual appearance
- Water content (%w/w) by Karl Fisher analysis
- Assay (% w/w) and related substances determination
- Colour of solution by UV spectrophotometry.

The results obtained for each test were used to assess the stability of the various salts and the base.

### Experimental procedures

### Materials

### Test Item Characterization, Sample Description

Six different salts of morphine-6-glucuronide were prepared from morphine-6-glucuronide base; the hydrobromide (HBr), sulphate (H₂SO₄), phosphate (H₃PO₄), hydrochloride (HCl), fumarate and maleate. The HBr salt was prepared by the method described in Example 2. The only difference was that after the 2-propanol slurry was filtered, the solid was then washed three times with diethyl ether, before drying under vacuum at room temperature. This additional step was employed to remove as much 2-propanol from the salt as possible.

The other inorganic salts (sulphate, phosphate, hydrochloride) were prepared in a similar way, i.e. by addition of the relevant acid to a cooled stirring suspension of morphine-6-glucuronide base in methanol, trituration of the resultant solution with cooled 2-propanol to form a suspension, and then continuous stirring at low temperature. Filtration of the solid is followed by washing with diethyl ether, and then drying at room temperature under reduced pressure.

The maleate and fumarate were prepared by the addition of the desired acid, on stirring at room temperature, to an aqueous solution of morphine-6-glucuronide base until all material was dissolved. The solution was then freeze dried to produce the required solid.

The same batch of morphine-6-glucuronide base (Batch M01003) was used to prepare each salt. This batch had been synthesised and tested to confirm identity, chemical and microbiological purity.

All salts prepared were tested to confirm appearance, assay (%w/w) by HPLC, confirmation of presence of correct counter ion, water content (%w/w) by Karl Fisher analysis, residual solvent analysis by GC and determination of colour of solution by measurement of UV absorbance of a 5 %w/v solution at 420 nm.

### Description of Salts of Morphine-6-glucuronide:

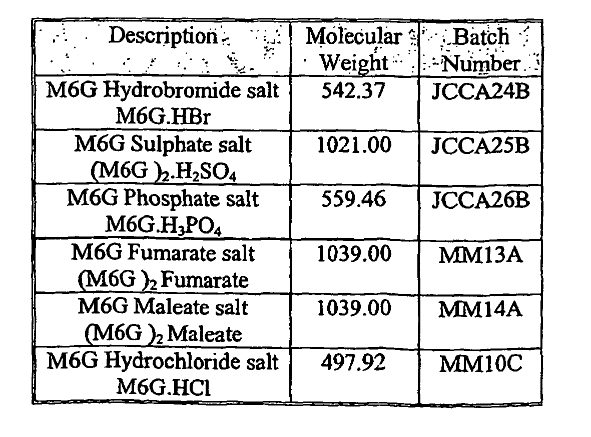

Description of Reference substances used in Testing of Salts:

| Reference substance | Description | Batch number |
|---|---|---|
| HN-33169 | M6G | 401-2055 |
| HN-33177 | Synthetic impurity | 401-2052 |
| HN-75083 | Degradant | 401-2054 |
| HN-75076 | Degradant | 401-2044 |
| HN-67003 | Degradant | 401-2058 |
| Morphine sulphate pentahydrate | Degradant | 40IK1192 |

Each test material was stored between 2-8°C prior to placing on stability. Each material was sub-divided into 900mg aliquots, transferred to brown opaque HDPE plastic bottles and flushed with Argon prior to sealing. Sufficient samples were provided for each time point as well as spares for each storage condition. The samples were placed in appropriate incubators previously commissioned at storage conditions 25°C/60%RH, 40°C/75%RH and 60°C.

The reference materials were stored under secure conditions at -20°C or below until required for testing.

### Methods

The samples were stored for analysis according to the following table:

| Storage Condition | Initial | 1 month | 3 months |
|---|---|---|---|
| 25°C/60% RH | X | X | X |
| 40°C/75%RH | | X | X |
| 60°C | | X | - |

X = Appearance, Water content by Karl Fischer analysis, Assay and Related Substances and Colour by UV /Vis spectrophotometry.

### Testing Procedures

### Test for identity Content and Impurities

Testing was performed in duplicate (2 x 25mg) in accordance with a stability indicating HPLC assay method. The assay results were reported as M6G as is, M6G as the anhydrous, solvent free material and the anhydrous solvent free material corrected for the salt form using the relevant conversion factor.

### Water Content by Karl Fisher Analysis

Water content was determined in duplicate on an aliquot of equilibrated material (approximately 100 mg) using a Tritrino 720 KFS Titrator.

### Colour by Visible Spectrophotometry

A 5% w/v solution of test material was prepared in water and the absorbance measured at 420nm in a 1cm silica cell using a Unicam UV4 Visible/UV spectrophotometer.

### Results

These are shown in Tables 2-4.

### Discussion

On storage for 3 months at 25°C/60%RH the hydrobromide, hydrochloride, phosphate and base remain as white crystalline solids, the other salts showing varying degrees of colouration. However on storage at 40°C/75%RH over the same period, all the salts (except the hydrobromide) plus the base show signs of becoming yellow in appearance. The change in appearance is reflected in the results for colour of solution, which increases in value as the yellow colour of the solid becomes more intense.

The general trend in moisture content is that the higher the storage humidity the greater the moisture content of the samples. The exception however is the base, where the moisture content is reasonably consistent regardless of storage condition. Of the salts the largest change in moisture content is with the phosphate (increase of around 8% at 40°C/75%RH compared to initial).

Review of the 3 month assay data shows some interesting trends. The most stable materials (based on %w/w assay) are the hydrobromide, base and phosphate. It should be noted that the reason that the phosphate assay values are high throughout the study (around 110%± 5%), is that there were some problems in the preparation of this salt. These issues resulted in the material being present as a mixture of phosphate/base in a ratio of approximately 10.8:1. The maleate and fumarate show a drop in assay of around 10% after 3 months at 40°C/75%RH compared to the initial values. Interestingly the hydrochloride shows a small decrease in assay after 3 months storage at 25°C/60%RH (around 6% compared to initial), however a dramatic reduction at 3 months storage at 40°C/75%RH (approx 34% decrease compared to initial). This reduction is in fact more than that seen with the sulphate salt, which from the 1-month data alone was thought to be the most unstable salt. The low assay value seen at 3months 40°C/75%RH, may be linked to the breakdown of the crystal form at high humidity resulting in a high degree of degradation. This degradation is reflected in the amount of degradation products seen in this sample (total of around 54.5%)

Even after 3 months storage at 40°C/75%RH there is basically no increase in the amount of degradation products in the hydrobromide salt as measured by HPLC. At the same conditions, there is an increase of approximately 3% in the amount of degradation products in the base. The levels of degradation are similar for the fumarate and maleate, slightly less for the phosphate. The least stable salts are the sulphate and the hydrochloride, with some indication that the hydrochloride is more stable than the sulphate at 25°C/60%RH, but the reverse being the case at 40°C/75%RH.

### Conclusion

The results obtained indicate that the hydrobromide salt appears more stable than all other salts and the base. An overall review of the data suggests the following order of stability:
Hydrobromide>base>>phosphate/maleate/fumarate>sulphate/hydrochloride

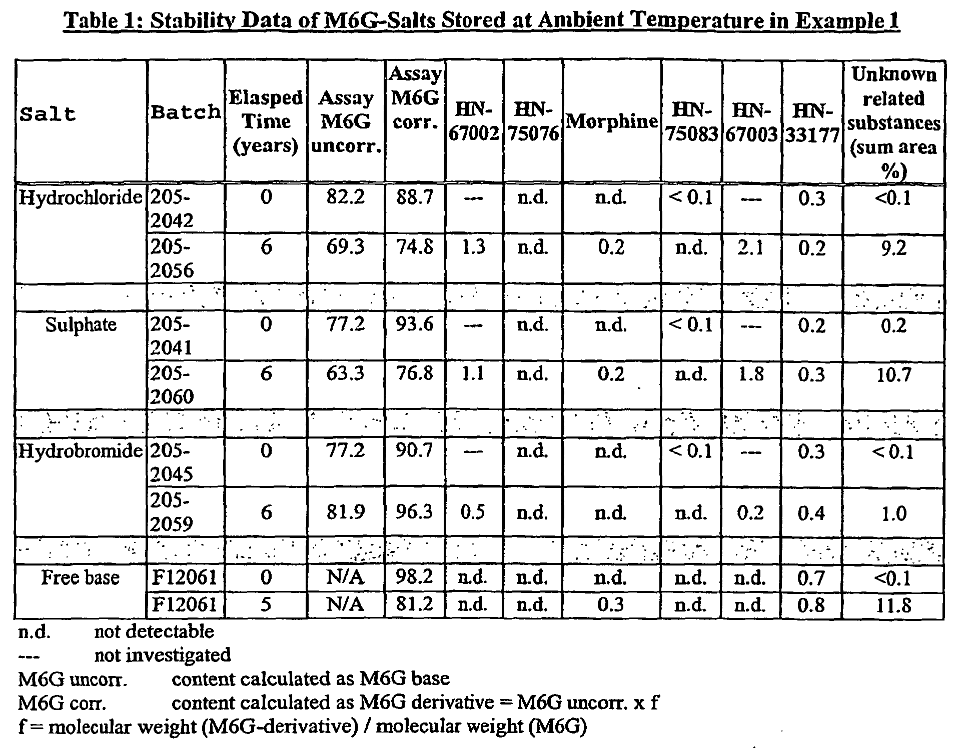

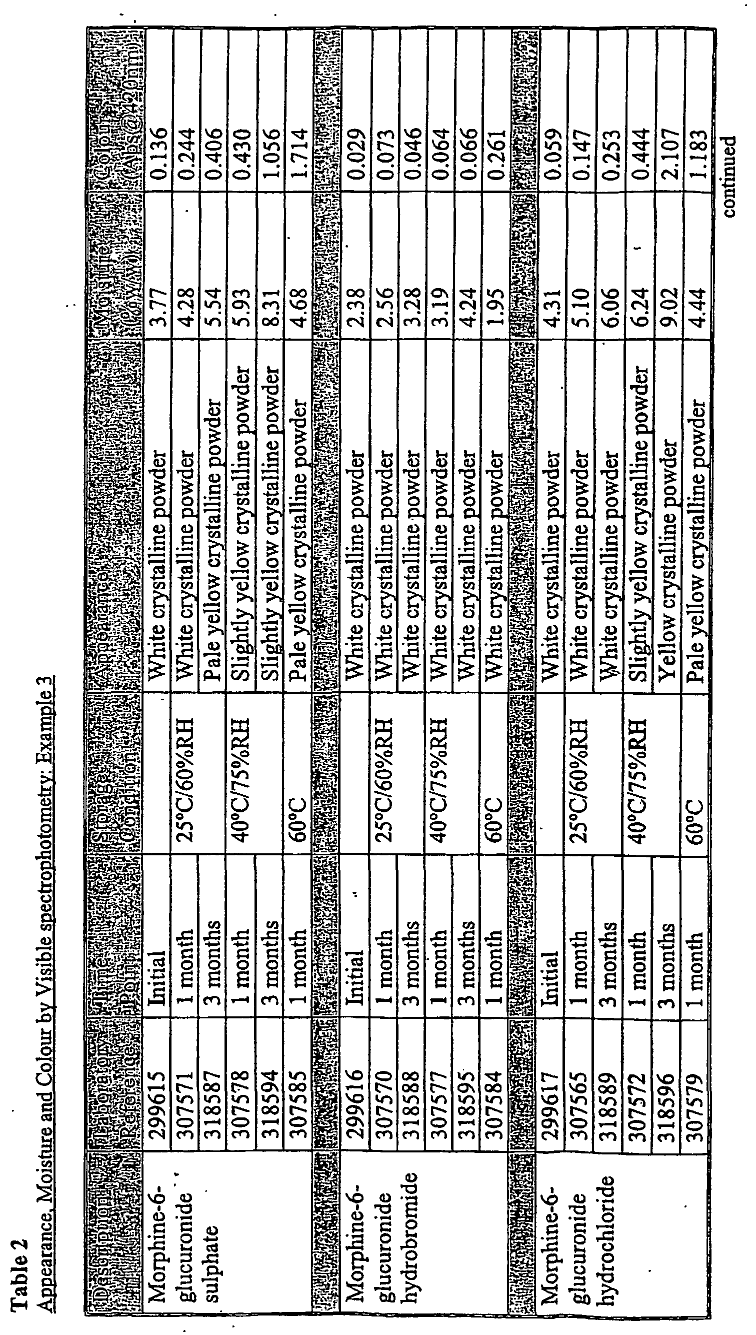

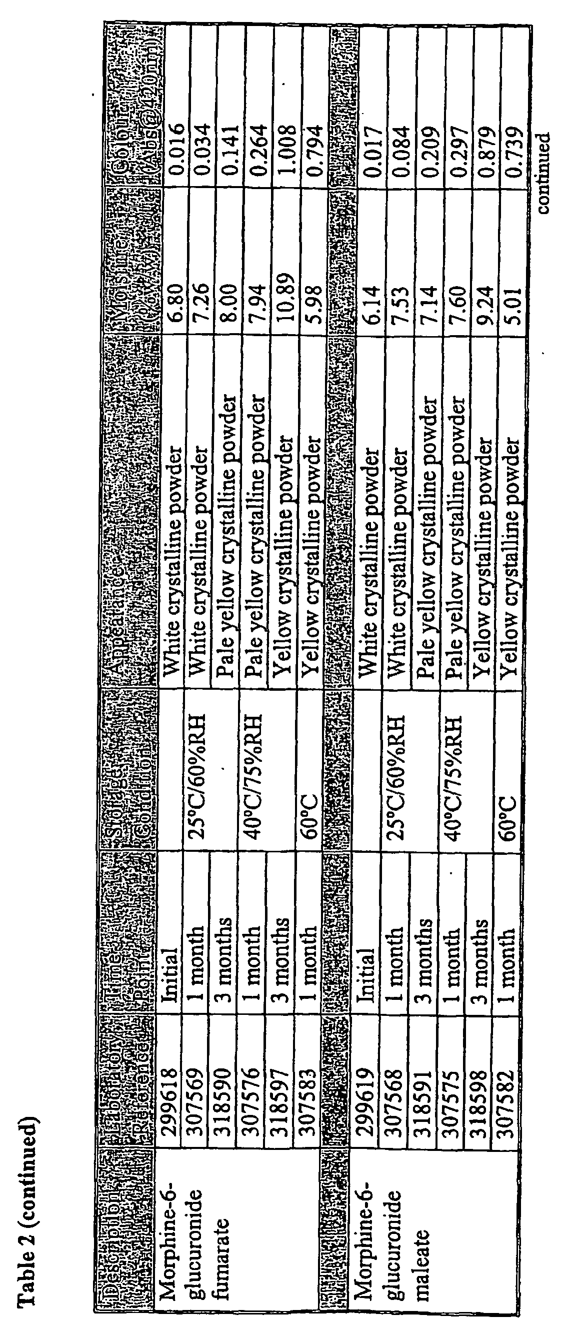

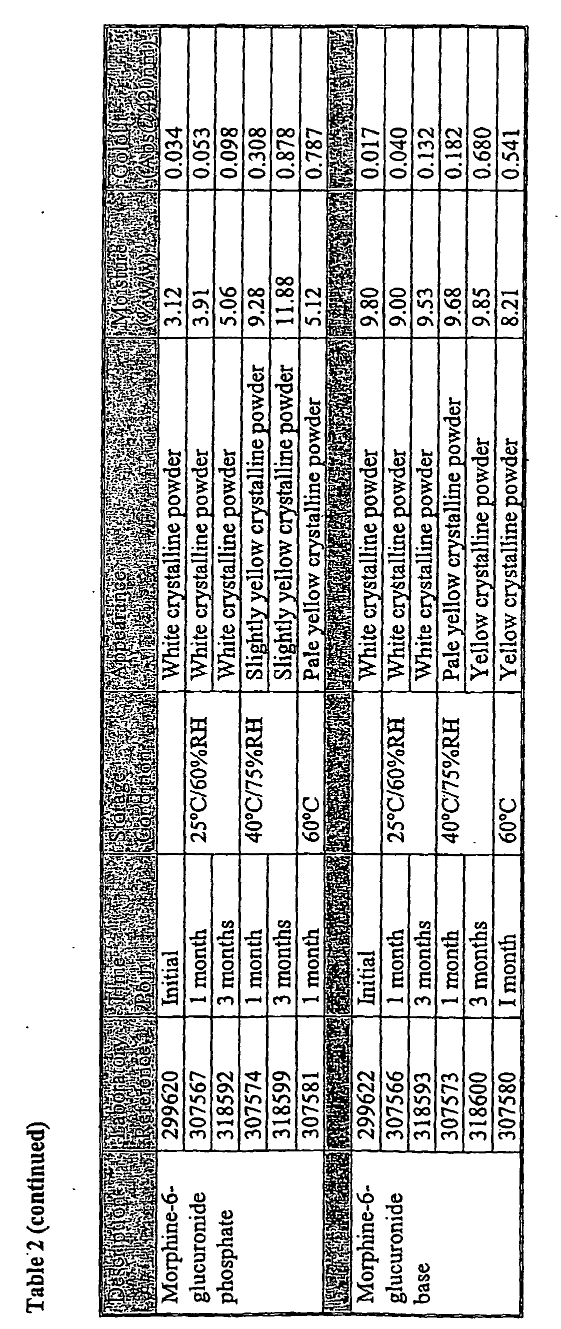

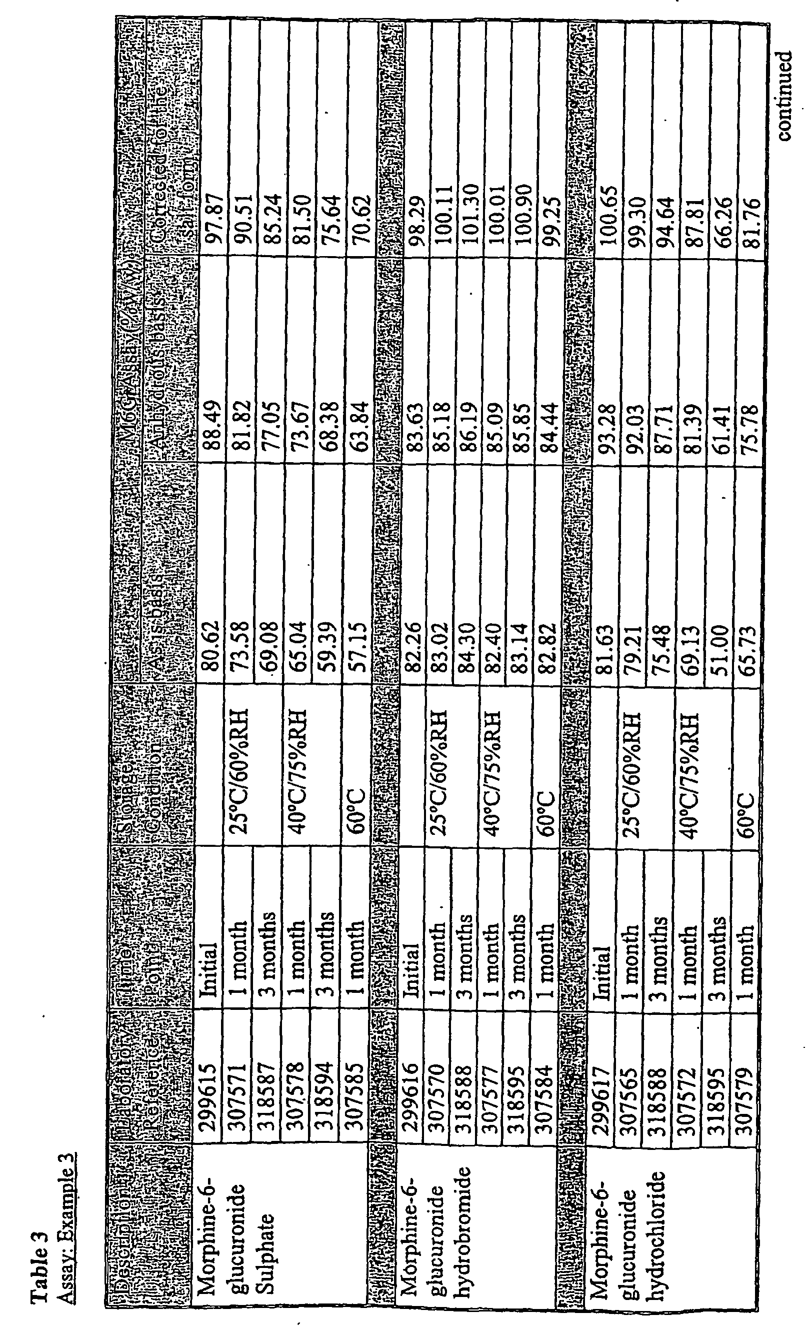

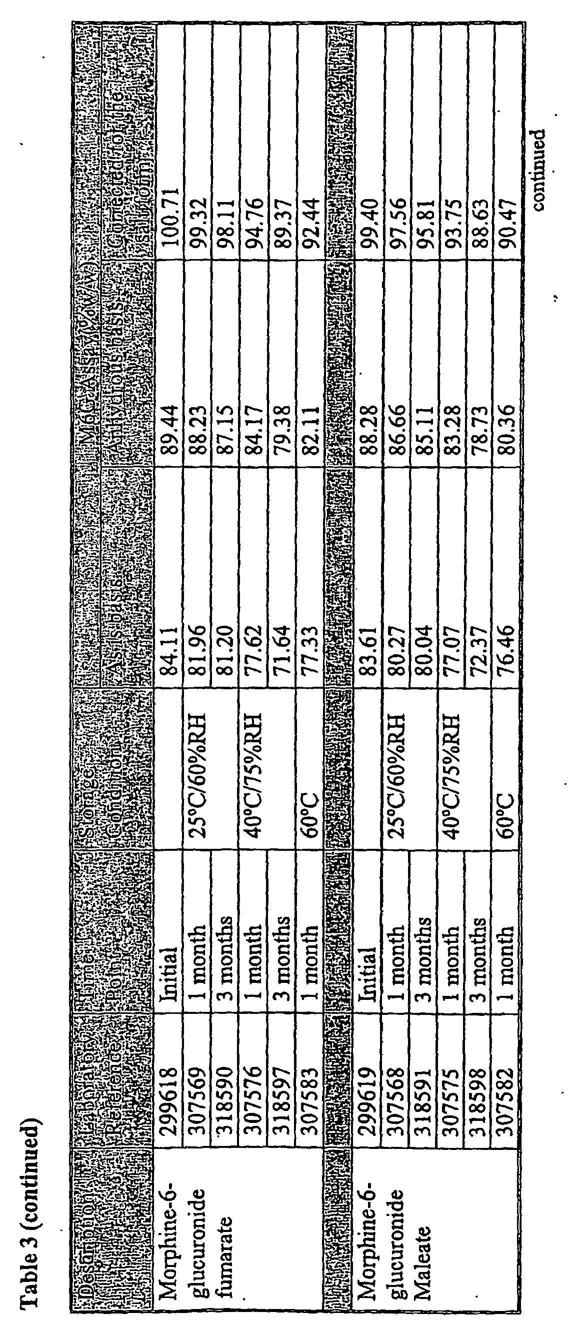

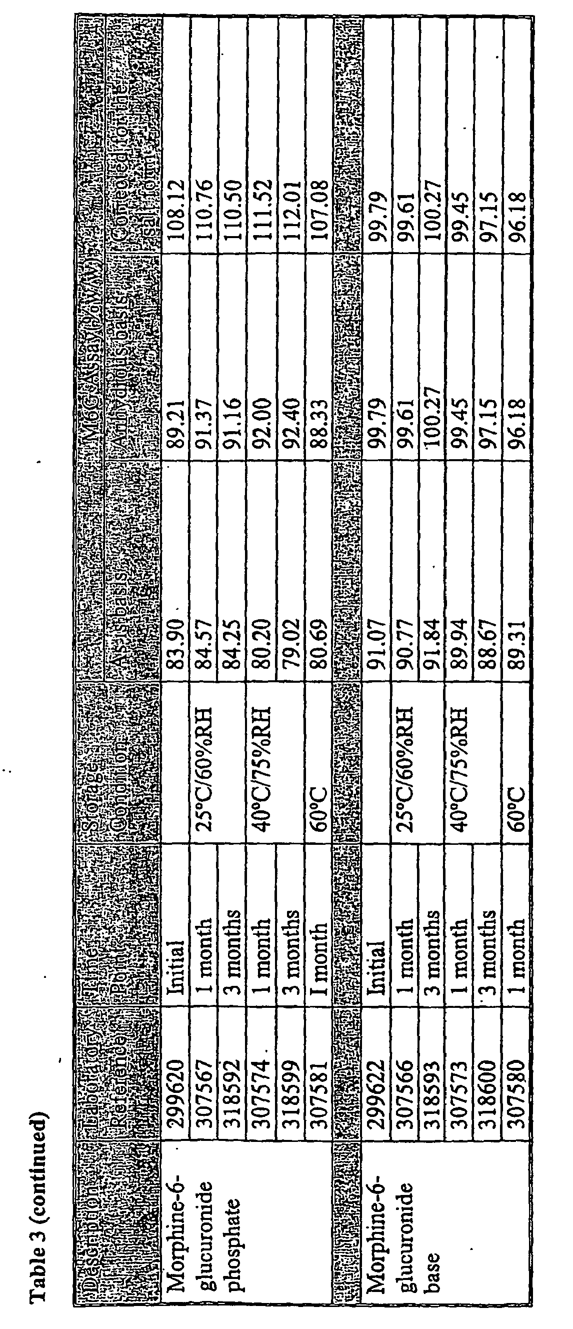

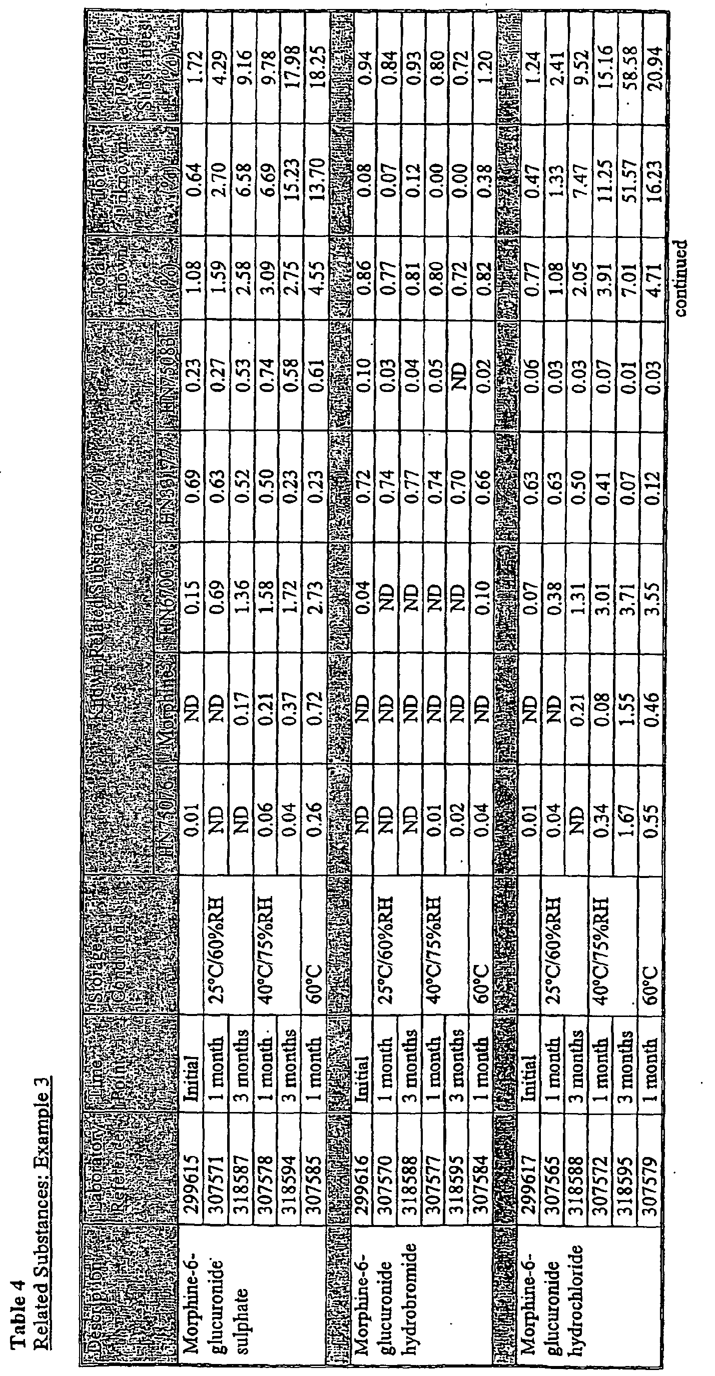

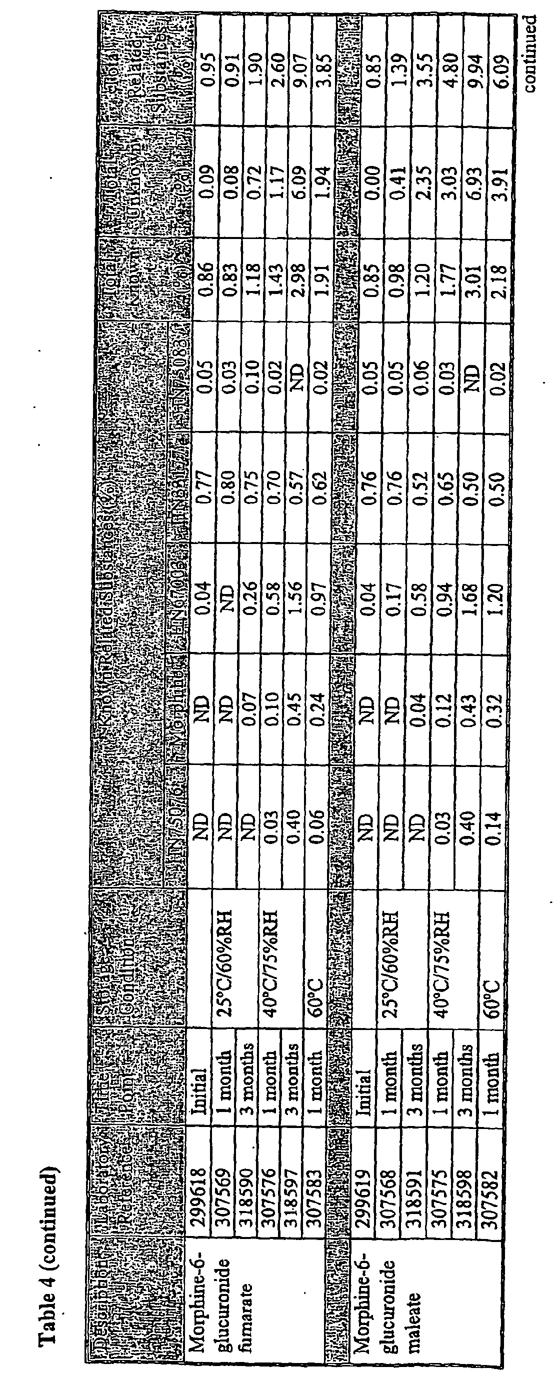

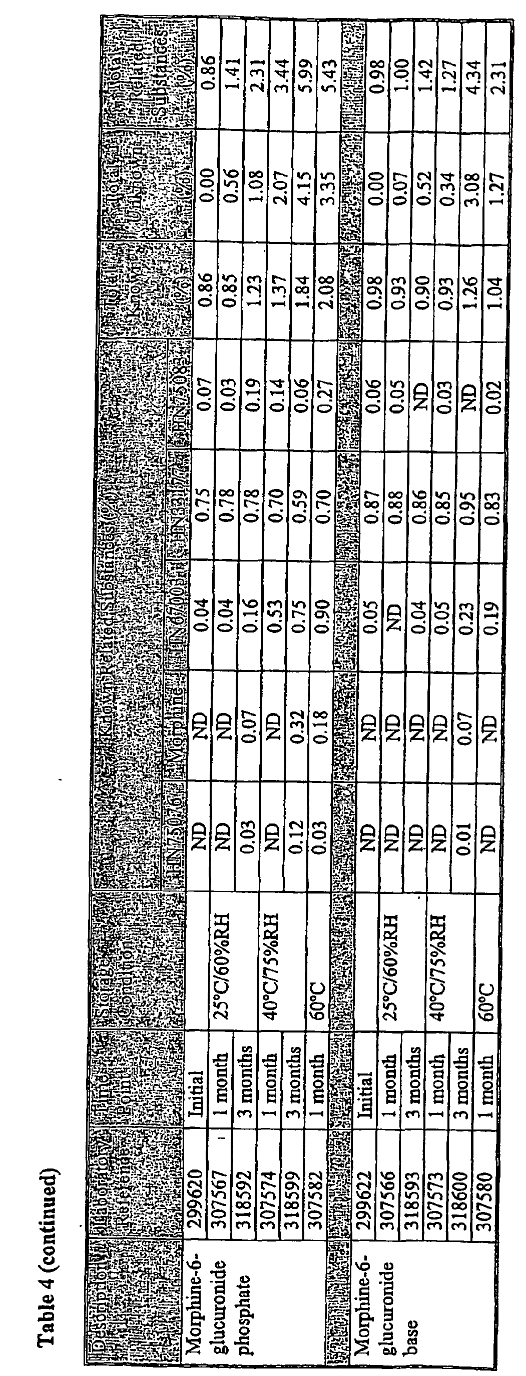

## Claims

1. A hydrobromide salt of morphine-6-β-D-gluCuronide (M6G.HBr).

2. A pharmaceutical composition comprising an analgesically effective amount of M6G.HBr together with a pharmaceutically acceptable carrier, excipient, or diluent.

3. A pharmaceutical composition comprising an amount of M6G.HBr effective for the treatment of breathlessness in a subject with advanced cancer, together with a pharmaceutically acceptable carrier, excipient, or diluent.

4. M6G.HBr for use as a medicament.

5. Use of M6G.HBr in the manufacture of a medicament for the treatment of pain.

6. Use according to claim 5 for the treatment of moderate to severe pain in acute or chronic conditions.

7. Use of M6G.HBr in the manufacture of a medicament for the treatment of breathlessness in a subject with advanced cancer.

8. A method of making M6G.HBr which comprises:
(i) contacting a hydrogen bromide solution with a solution of M6G in methanol;
(ii) contacting the solution resulting from step (i) with an organic solvent to precipitate M6G.HBr; and
(iii) isolating M6G.HBr precipitated in step (ii).

9. A method according to claim 8, in which the solutions and solvent are at -15°C, or below.

10. A method according to claim 8 or 9, which further comprises washing the precipitated M6G.HBr to minimise the amount of organic solvent present.

11. A method according to claim 10, in which the precipitated M6G.HBr is washed with diethyl ether.

12. A method according to any of claims 8 to 11, in which the organic solvent of step (ii) is 2-propanol.

## Patentansprüche

1. Hydrobromidsalz von Morphino-6-β-D-glucuronid (M6G.HBr).

2. Pharmazeutische Zusammensetzung, die eine analgetisch wirksame Menge von M6G.HBr zusammen mit einem pharmazeutisch akzeptablen Träger, Bindemittel oder Verdünnungsmittel umfasst.

3. Pharmazeutische Zusammensetzung, die eine Menge von M6G.HBr, die zur Behandlung von Kurzatmigkeit bei einer Person mit fortgeschrittener Krebserkrankung wirksam ist, zusammen mit einem pharmazeutisch akzeptablen Träger, Bindemittel oder Verdünnungsmittel umfasst.

4. M6G.HBr zur Verwendung als Medikament.

5. Verwendung von M6G.HBr zur Herstellung eines Medikamentes zur Behandlung von Schmerzen.

6. Verwendung nach Anspruch 5 zur Behandlung von mäßigen bis starken Schmerzen bei akuten oder chronischen Krankheiten.

7. Verwendung von M6G.HBr zur Herstellung eines Medikaments zur Behandlung von Kurzatmigkeit bei einer Person mit fortgeschrittener Krebserkrankung.

8. Verfahren zur Herstellung von M6G.HBr, das die folgenden Schritte umfasst:
(i) Inkontaktbringen einer Bromwasserstofflösung mit einer Lösung aus M6G in Methanol;
(ii) Inkontaktbringen der aus Schritt (i) hervorgehenden Lösung mit einem organischen Lösungsmittel zum Präzipitieren von M6G.HBr; und
(iii) Isolieren des im Schritt (ii) präzipitierten M6G.HBr.

9. Verfahren nach Anspruch 8, bei dem die Lösungen und das Lösungsmittel bei -15°C oder darunter liegen.

10. Verfahren nach Anspruch 8 oder 9, das ferner das Waschen des präzipitierten M6G.HBr umfasst, um die Menge des anwesenden organischen Lösungsmittels gering zu halten.

11. Verfahren nach Anspruch 10, wobei das präzipitierte M6G.HBr mit Diethylether gewaschen wird.

12. Verfahren nach einem der Ansprüche 8 bis 11, wobei das organische Lösungsmittel aus Schritt (ii) 2-Propanol ist.

## Revendications

1. Sel bromhydrate de morphine-6-β-D-glucuronide (M6G.HBr).

2. Composition pharmaceutique comprenant une quantité efficace en tant qu'analgésique de M6G.HBr, avec un véhidule, excipient ou diluant pharmaceutiquement acceptable.

3. Composition pharmaceutique comprenant une quantité efficace de M6G.HBr pour le traitement de l'essoufflement chez un sujet atteint d'un cancer avancé, avec un véhicule, un excipient ou un diluant pharmaceutiquement acceptable.

4. M6G.HBr à utiliser en qualité de médicament.

5. Utilisation de M6G.HBr dans la fabrication d'un médicament pour le traitement de la douleur.

6. Utilisation selon la revendication 5, pour le traitement d'une douleur de modérée à sévère dans des conditions aiguës ou chroniques.

7. Utilisation de M6G.HBr dans la fabrication d'un médicament pour le traitement de l'essoufflement chez un sujet atteint d'un cancer avancé.

8. Procédé de fabrication de M6G.HBr qui comprend :
(i) mettre une solution de bromure d'hydrogène en contact avec une solution de M6G dans du méthanol;
(ii) mettre la solution résultant de l'étape (i) en contact avec un solvant organique pour précipiter le M6G.HBr; et
(iii) isoler le M6G.HBr précipité à l'étape (ii).

9. Procédé selon la revendication 8, dans lequel les solutions et le solvant sont à ― 15°C ou moins.

10. Procédé selon la revendication 8 ou 9, qui comprend en outre laver le M6G.HBr précipité pour minimiser la quantité de solvant organique présent.

11. Procédé selon la revendication 10, dans lequel le M6G.HBr précipité est lavé avec de l'éther diéthylique.

12. Procédé selon l'une quelconque des revendications 8 à 11, dans lequel le solvant organique de l'étape (ii) est du 2-propanol.
